Europäisches Patentamt

European Patent Office

Office européen des brevets

(11) Veröffentlichungsnummer: **0 377 174**
**A2**

(12) EUROPÄISCHE PATENTANMELDUNG

(21) Anmeldenummer: **89123659.8**

(22) Anmeldetag: **21.12.89**

(51) Int. Cl.⁵: **A61M 11/00**

(30) Priorität: **22.12.88 DE 3843317**

(43) Veröffentlichungstag der Anmeldung:
**11.07.90 Patentblatt 90/28**

(84) Benannte Vertragsstaaten:
**AT BE CH DE ES FR GB GR IT LI LU NL SE**

(71) Anmelder: **Fischer, Jürgen L.**

**CH-Tegna(CH)**

Anmelder: **Büsselmann, Manfred**
**21 Rue Racine**
**F-75006 Paris(FR)**

(72) Erfinder: **Ronge, Georg**
**Postfach 722**
**D-8000 München 33(DE)**

(74) Vertreter: **Vossius & Partner**
**Siebertstrasse 4 P.O. Box 86 07 67**
**D-8000 München 86(DE)**

(54) Verfahren und Vorrichtung zum Zerstäuben und Vernebeln von flüssigen Stoffen.

(57) Das Verfahren und die Vorrichtung zum Zerstäuben und Vernebeln von flüssigen Stoffen dient insbesondere zum Herstellen von Nebeln für Inhalationszwecke. Dabei wird der zu zerstäubende flüssige Stoff derart hoch komprimiert, daß sein Volumen verringert wird und dann explosionsartig in die normale Atmosphäre entlassen wird. Durch den inneren hohen Druck wird der flüssige Stoff in kleinste Teilchen zerrissen.

EP 0 377 174 A2

## Verfahren und Vorrichtung zum Zerstäuben und Vernebeln von flüssigen Stoffen

Die Erfindung betrifft ein Verfahren und eine Vorrichtung zum Zerstäuben und Vernebeln von flüssigen Stoffen zu Inhalationszwecken.

Bei den bisher bekannten Verfahren zur Aerosol-Herstellung werden Aerosole mittels Luftstrom, Gebläsen oder durch Zentrifugal-Zerstäuber hergestellt. Die bekannten Inhalationsverfahren werden eingesetzt, um die Essenz einer flüssigen Substanz nach ihrer Umwandlung in Aerosol durch Einatmung an die Oberflächen der Bronchien und Bronchiolen zu bringen, wo die zerstäubte Flüssigkeit ihre therapeutische Wirkung ausübt.

Ziel der vorliegenden Erfindung ist es, ein neuartiges Verfahren und eine Vorrichtung bereitzustellen zum Zerstäuben und Vernebeln von flüssigen Stoffen, wobei die Teilchen durch Inhalation besonders gut aufgenommen werden.

Das erfindungsgemäße Verfahren und die Vorrichtung zeichnen sich aus durch die Merkmale der Patentansprüche.

Nach dem erfindungsgemäßen Verfahren und der Vorrichtung wird der zu vernebelnde Flüssigkeits-Körper mit einem Druck von 300 bis 800 x $10^5$ Pascal so stark komprimiert, daß sein Volumen verringert wird. Anders als bei Gasen gelingt solch eine volumenändernde Kompression nur durch sehr hohe Drücke. Wird dieser so komprimierte Flüssigkeits-Körper in die normale Atmosphäre von $10^5$ Pascal (1 bar) entlassen, so explodiert der Flüssigkeits-Körper durch seinen inneren hohen Druck nach allen Richtungen hin in kleinste Teile und zerstäubt zu einem sehr feinen Nebel.

Der zu zerstäubende flüssige Stoff enthält vorzugsweise Vitamine, insbesondere Vitamin A, B, C und/oder E und gegebenenfalls zusätzlich Lecithin. Die genannten Vitamine bzw. das Lecithin sind in geeigneter Dosierung in einer Flüssigkeit, wie Wasser, vorzugsweise Öl gelöst.

Erfindungsgemäß können vor allem auch Flüssigkeiten hoher Viskosität, insbesondere Öle, die von den bisher bekannten Verfahren überhaupt nicht ausreichend vernebelt werden konnten, in feinste Teilchen zerstäubt werden. Insbesondere können dadurch ölige oder ölgelöste Substanzen wie Vitamin A und E zerstäubt werden.

Vorzugsweise wird pflanzliches Öl, wie Erdnußöl, in dem Vitamine z.B. A und E enthalten sind, zerstäubt.

Vorteilhafterweise enthält der flüssige Stoff 0,5 bis 5 Gew.-% Vitamin A und/oder 0,5 bis 5 Gew.-% Vitamin E. Besonders bevorzugt ist eine Mischung von Erdnußöl mit 2,5 Gew.-% Vitamin-A-Acetat und 4 Gew.-% Vitamin-E-Tocoferol.

Insbesondere bevorzugt ist eine Mischung von Erdnußöl mit 1,25 Gew.-% Vitamin-A-Acetat und 0,5 Gew.- Vitamin-E-Tocoferol.

Erfindungsgemäß wird die zu zerstäubende Substanz mit einem Druck von 300 bis 800 x $10^5$ Pascal komprimiert. Der eingestellte Druck ist abhängig von dem zu zerstäubenden flüssigen Stoff. Oberhalb eines bestimmten Drucks erfolgt für die einzelnen Flüssigkeiten eine Zustandsänderung, d.h. das Volumen verringert sich. Flüssige Stoffe mit einer höheren Viskosität erfordern einen höheren Druck. Dieser für die Vernebelung der flüssigen Stoffe notwendige Druck beträgt für viskose Flüssigkeiten, z.B. organische Öle, zwischen 500 und 800 x $10^5$ Pascal. Für wäßrige Flüssigkeiten, z.B. für salzige Lösungen, wird ein Druck von 300 bis 500 x $10^5$ Pascal, vorzugsweise 430 x $10^5$ Pascal eingestellt.

Wird eine Flüssigkeit mit einem zu niedrigem Druck, z.B. mit 100 x $10^5$ Pascal komprimiert, so erfolgt nicht die explosionsartige Zerstäubung des Stoffes, statt dessen tritt wie bei der Dieseleinspritzanlage ein geschlossener Strahl der Flüssigkeit aus der Düse aus. Durch die Wahl des geeigneten Drucks, je nach Viskosität der Substanz-Flüssigkeit, entsteht statt des "Diesel"-Strahls der gewünschte Nebel.

Auch die Leitfähigkeit, z.B. eines salzhaltigen Flüssigkeitsstrahles, besorgt bei einer elektrischen Spannung zwischen Empfänger und Düse sofort einen Zusammenbruch der Spannung. Wird aber diese Flüssigkeit nach dem erfindungsgemäßen Verfahren zerstäubt, so besitzt der so vernebelte Flüssigkeitskörper nach Verlassen der Öffnung des Kompressionsraumes auch unmittelbar an der Austrittsöffnung keine Durchschlagsenergie, und eine Hochspannungselektrode, die unmittelbar vor der Öffnung eines Kompressionsraumes angeordnet ist, verliert bei der Vernebelung salzhaltiger Flüssigkeit keinerlei Spannung gegenüber dem geeigneten Kompressionsaggregat.

Mit dem erfindungsgemäßen Verfahren werden Teilchen mit einer Größe von etwa 0,5 bis 10 $\mu m^3$, vorzugsweise etwa 1 $\mu m^3$ erzeugt, d.h., daß ein $mm^3$ Flüssigkeit in eine Milliarde Teilchen zerrissen wird. Erfindungsgemäß weisen die erzeugten Teilchen ein Potential auf, das für alle Teilchen gleich ist, so daß sich die Teilchen im Nebel gegenseitig abstoßen. Das Potential der Teilchen, d.h. der zerstäubten Flüssigkeitströpfchen ist verschieden von dem Potential der Umgebung und die Tröpfchen sind vorzugsweise positiv geladen. Das erfindungsgemäße Verfahren kann solange durchgeführt werden, bis die gewünschte Menge eines flüssigen Stoffes zerstäubt ist. Gemäß einer bevorzugten Ausführungsform der Erfindung wird nachdem der zu zerstäubende flüssige Stoff komprimiert wurde, nur eine Teilmenge von etwa 0,25 ml in die normale Atmosphäre entlassen und dadurch zerstäubt, wobei dieser Vorgang mehrmals z.B. 200 mal wiederholt wird.

Die erfindungsgemäße Vorrichtung umfaßt einen Kompressions-Raum, der einem Druck von 300 bis 800 x 10^5 Pascal zum Komprimieren der Flüssigkeit standhält, mit einer oder mehreren kleinen Öffnungen, wobei bei einem bestimmten inneren Druck der Flüssigkeit von z.B. 600 bis 800 x 10^5 Pascal (1 bar) beim Austritt der Flüssigkeit durch diese Öffnung der Druck der Flüssigkeit im Inneren des Kompressionsraums nicht unter einen gewünschten Druck von z.B. 550 x 10^5 Pascal abfällt. Auf diese Weise wird die in dem Kompressionsraum eingegebene Flüssigkeit beim Austritt durch die feinen Öffnungen durch das hohe Druckgefälle in die gewünschten Nebelteilchen explosionsartig auseinandergerissen. Der Druck wird von einer Hochdruckpumpe, die die flüssigen Stoffe über eine Leitung zum Kompressionsraum leitet, erzeugt.

Erfindungsgemäß öffnen diese gesteuerten Düsen selbsttätig bei einem oberen Druck und schließen wieder selbsttätig bei einem unteren Druck. Die obere Grenze ist vorzugsweise zwischen 500 und 800 x 10^5 Pascal und die untere Grenze ist vorzugsweise zwischen 300 und 600 x 10^5 Pascal einstellbar.

Vorzugsweise ist die Größe des Kompressionsraumes einstellbar, wodurch die Menge der abgegebenen zu zerstäubenden Flüssigkeit bei einem fest eingestellten oberen und unteren Druckwert veränderbar ist.

Gemäß einer bevorzugten Weiterbildung der Erfindung ist die steuerbare Düse an einem Nebeltopf angeordnet, so daß die zerstäubte Flüssigkeit in den Nebeltopf entlassen wird. Der Nebeltopf ist vorzugsweise ein Zylinder mit einem Boden- und Deckelteil. Darüber hinaus sind am Nebeltopf eine Zuluftöffnung und eine Auslaßöffnung vorgesehen, die vorzugsweise jeweils mit einem Schalldämpfer ausgestattet sind. Der Nebeltopf dient zum einen der gleichmäßigen Verteilung der zerstäubten Flüssigkeit und zum anderen der Schallisolation der Düsenanordnung. Bevorzugt ist an der Auslaßöffnung ein geeignetes Mundstück bzw. ein Atemtrichter angeordnet, über das die zerstäubte Flüssigkeit inhaliert werden kann.

Bei einer weiteren Ausführungsform ist in dem Mundstück ein Einweg-Ventil angeordnet, das nur öffnet, wenn Luft aus dem Nebeltopf angesaugt wird. In der anderen Richtung sperrt es und verhindert somit, daß Luft von außen, z.B. die ausgeatmete Luft in den Nebeltopf gelangen kann. Dadurch wird in vorteilhafter Weise vermieden, daß Verunreinigungen in den Nebeltopf gelangen.

Das erfindungsgemäße Verfahren und die Vorrichtung hat insbesondere den Vorteil, daß zur Inhalation ein feinster Nebel erzeugt wird, mit dem die für die Therapie geeignete Substanz unmittelbar eingeatmet werden kann. Es können Stoffe der verschiedensten Art, selbst hochviskose organische Öle und die in ihnen gelösten Substanzen, bis zu den Oberflächen der Alveolen, der Lungenbläschen, gebracht werden. Von diesen werden sie aufgenommen, resorbiert, gewissermaßen "verdaut" und gelangen somit unmittelbar in das arterielle Blut.

Die Lunge ist ein ausgezeichnetes "Verdauungsorgan" des lebendigen Organismus. In gleicher Weise wie die riesigen Oberflächen der Blätter von Pflanzen gestattet sie die unmittelbare Aufnahme von in der Luft befindlichen Stoffen. Dies gestattet die Möglichkeit bei vollständiger Umgehung des gesamten Verdauungstraktes, Stoffe in die arteriellen Blutbahnen zu verbringen, die so an jedes Organ und an jede Stelle des Organismus transportiert werden können. Durch die Inhalation dichtester, konzentrierter Nebel der flüssigen Substanzen wird erreicht, daß in kurzer Zeit, z.B. schon nach einigen Atemzügen etwa in 1 Minute, wirkungsvolle Mengen der Substanz in die Alveolen und dann unmittelbar in den Blutkreislauf transportiert werden können.

Die vorliegende Erfindung hat den Vorteil, daß durch die Feinheit des Nebels die Tröpfchen unvergleichlich lungengängig sind, die Dichte des Nebels beliebig gesteuert werden kann und der Menge der Flüssigkeit, die vernebelt werden soll, keine praktischen Grenzen gesetzt sind.

Die vorliegende Erfindung zeichnet sich insbesondere durch den auftretenden technischen Effekt aus, d. h., daß zwischen den zerstäubten Flüssigkeitströpfchen einerseits und den Lungenalveolen andererseits aufgrund der unterschiedlichen Potentiale eine besondere Anziehung (Affinität) besteht. Aufgrund dieser Anziehung werden die Tröpfchen von den Alveolen ausgezeichnet aufgenommen. Vorzugsweise sind die Tröpfchen positiv geladen, während die Zellen der Lungenalveolen negativ geladen sind. Die Wirkung der Anziehungskraft ist umso größer, je kleiner die Tropfen sind. Zum einen ist die Beweglichkeit der Tropfen größer und zum anderen ist die Ladung pro Masseeinheit größer.

Mit dem erfindungsgemäßen Verfahren ist es möglich, in 1 Minute 15 ml der zu zerstäubenden flüssigen Stoffe in die Alveolen zu bringen, wo sie aufgenommen und resorbiert werden. Ein beachtlicher Teil der insgesamt inhalierten Sub stanz bleibt dabei an den Bronchialgefäßen haften, wird dort aufgenommen, und ein geringer Teil verläßt als sehr feiner, rauchartiger "Dampf" die Lunge wieder während der Ausatmung.

Die "alternative" Verabreichung von Stoffen über den Verdauungstrakt mit seinen substanzverändernden Säften und Enzymen - Magen, Bauchspeicheldrüse, Leber, Galle -, wird umgangen, so daß die Substanz "ungestört" und unverändert an alle Organe und Systeme des Gesamtorganismus transportiert wird. Sollen die obengenannten Verdauungswege umgangen werden, so bietet sich bisher lediglich die Möglichkeit der Injektion. Diese Applikationsmethode verbietet bekanntermaßen das Einbringen öliger Substanzen. Mit dem erfindungsgemäßen Verfahren können insbesondere öllösliche Substanzen ungestört dem Organismus zugeführt werden. Beispielsweise können Vitamine, z.B. A und E in möglichst diffuser, hauptsächlich molekularer Form an innere Organe, besonders an Epithele und Bindegewebe, und an die nervalen und zentral-nervalen Systeme geführt werden. Krankheitsherde im Organismus können durch die Inhalation der erfindungsgemäß bereitgestellten Nebel von pflanzlichem Öl, z.B. Erdnußöl, mit den darin gelösten Vitaminen A und E therapeutisch behandelt werden. In Versuchen hat sich die Wirksamkeit des erfindungsgemäßen Verfahrens und der Vorrichtung bestätigt. Das erfindungsgemäße Verfahren ist ebenso geeignet zur Herstellung eines Inhalationsnebels für die Behandlung der Bronchien und der Bronchiolen, bei asthmatischen Erkrankungen und anderen sonst schwierig zu therapierenden Leiden. Das Verfahren ist weiterhin einsetzbar für die Inhalation von Stoffen, die eine wesentliche Steigerung des Wohlbefindens, der Schmerzfreiheit, der Schlaffestigkeit und eine Steigerung der körperlichen und geistigen Leistungen erreichen. Das Verfahren wirkt zentral am ganzen Organismus. Das erfindungsgemäße Verfahren eignet sich auch hervorragend zum Zerstäuben und Vernebeln von wasserlöslichen Substanzen in Wasser. Das Gemisch kann zum Zwecke der Inhalation vernebelt werden, z.B. Meersalzlö sungen oder Solen in Badeorten, in der Sauna und zur Intensivierung des Salinen-Effektes.

Die Erfindung wird nachstehend anhand eines Beispiels und der Zeichnung näher erläutert.

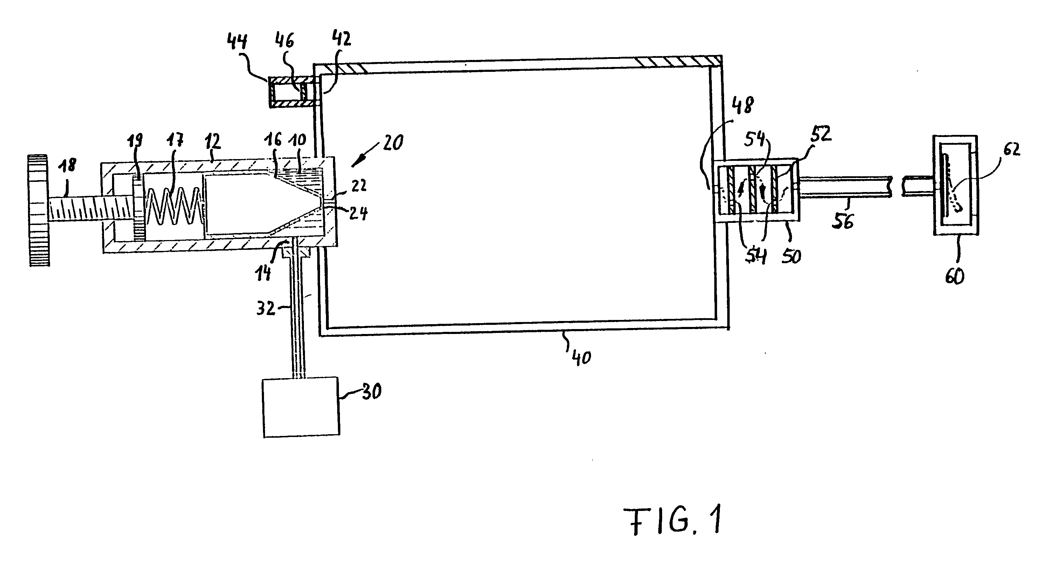

Figur 1 zeigt eine bevorzugte Ausführungsform der erfindungsgemäßen Vorrichtung.

Die dargestellte bevorzugte Ausführungsform umfaßt einen Kompressionsraum 10 mit einer selbstgesteuerten Düsenanordnung 20, 22, 24. Die zu vernebelnde Flüssigkeit wird über ein Hochdruckrohr 32 aus der Hochdruckpumpe 30 befördert. Diese Pumpe kann eine Kolben- oder Zahnradpumpe sein. Der Kompressionsraum oder die Kammer 10 ist in einem Druckgefäß 12 angeordnet, wobei dieser über einen Kanal 14 mit dem Hochdruckrohr 32 in Verbindung steht. Die Kammer 10 hat in dem gezeigten Beispiel eine zylindrische Form und deren Abgrenzung wird gebildet durch einen Kolben 16, der eine konische Form hat und eine Öffnung 22 des Druckgefäßes 12 schließt. Der Kolben 16 ist in dem Gehäuse 12 beweglich und steht unter dem Einfluß einer wirkenden Kraft, z.B. einer Feder 17 und einer Schraube 18, durch welche die Feder reguliert werden kann. Die transportierte Flüssigkeit erreicht so einen hohen Druck, der durch die Feder 17 reguliert wird, gehalten durch eine Platte 19 und den Kolben 16, so daß die wirkende Kraft der Feder 17 den Kolben 16 gegen die Öffnung 22 des Druckgefäßes drückt. Gelangt jetzt die im Raum 10 hochkomprimierte Flüssigkeit durch die Öffnung 22 nach außen, so vollzieht sich der explosionsartige Prozeß, hervorgerufen durch den Druckunterschied der in der Kammer 10 herrschenden Kompression und dem hinter der Öffnung 22 vorgefundenen Außendruck. Vorzugsweise hat die Öffnung 22 einen Durchmesser zwischen 0,1 und 0,25 mm, während der in dem Druckgefäß 12 wirkende Druckkolben 16 einen Durchmesser von 6 mm hat. Ist der Kolben größer, so kann auch die Öffnung 22 größer gewählt werden.

Es ist möglich, den begrenzten Raum 10 unmittelbar an der Pumpe 30 anzubringen. Mit Drücken von 300 bis 800 x $10^5$ Pascal oder noch größeren Drücken wird die austretende Flüssigkeit explosionsartig zerstäubt. Der entstehende Nebel ist so homogen und fein, daß keine Abfilterung größerer Partikel erforderlich ist. Eine größere Anzahl der Öffnungen 22 in Verbindung mit dem beweglichen Kolben und seiner schließenden Fläche 24 ist ebenfalls einsetzbar. Jedoch sollten die Querschnitte einer größeren Anzahl von Öffnungen zusammen nicht größer sein als der Öffnungsquerschnitt der einen Öffnung.

Weiterhin umfaßt die Vorrichtung einen Nebeltopf 40 in der Form eines Hohlzylinders mit einem aufsteckbaren Deckel- und Bodenteil. Die Düse 20 ist so am Bodenteil des Nebeltopfs 40 angeordnet, daß die zerstäubte Flüssigkeit in den Nebeltopf gelangt. Der Nebeltopf besteht vorzugsweise aus einem Kunststoff wie Acryl und hat eine Wanddicke von etwa 1 cm. Dadurch, daß das Deckel- und Bodenteil leicht abnehmbar und wieder aufsteckbar ist, kann der Nebeltopf ohne Schwierigkeiten gereinigt werden.

In der Nähe des Bodenteils bzw. am Bodenteil ist eine Zuluftöffnung 42 und in der Nähe des Deckelteils bzw. am Deckelteil ist eine Auslaßöffnung 48 vorgesehen. An der Zuluftöffnung 42 ist ein erster Schalldämpfer 44 angeordnet, der im Innern unter anderem einen Staubfilter 46 aufweist und zum einen der Luftfilterung und zum anderen der Schallisolation dient. An der Auslaßöffnung 48 ist ein zweiter Schalldämpfer 50 angeordnet, der ebenfalls zur Schallisolation dient. Durch die Schalldämpfer kann in vorteilhafter Weise die von der steuerbaren Düse ausgehenden Geräusche gedämpft werden. Der zweite Schalldämpfer 50 besteht im wesentlichen aus einem Hohlzylinder, dessen Durchmesser kleiner ist als der Durchmesser des Nebeltopfs 40, dessen Boden- und Deckelteil jeweils eine Öffnung haben. Im Innern des Hohlzylinders sind weitere scheibenförmige Blenden angeordnet, die jeweils eine kleine Öffnung aufweisen. Die Öffnungen im Boden- und Deckelteil sowie die Öffnungen in den scheibenförmigen Blenden sind bezogen auf die Mittenachse des Schalldämpfers gegeneinander versetzt, so daß die Luft, die aus dem Nebeltopf 40 durch den Schalldämpfer 50 nach außen geleitet wird, nicht auf einem geraden Weg sondern auf einem gewundenen Weg nach außen geleitet wird. Am Ausgang des Schalldämpfers 50 ist über einen Schlauch 56 ein Mundstück 60 angeschlossen. Im Innern des Mundstücks 60 ist ein Ventil 62 eingebaut, das in einer Richtung öffnet und zwar wenn die Luft aus dem Nebeltopf 40 zum Mundstück geleitet wird und in der entgegengesetzten Richtung schließt. Dadurch wird in vorteilhafter Weise vermieden, daß Außenluft z.B. die ausgeatmete Luft vom Mundstück zurück in den Nebeltopf gelangt.

**Ansprüche**

1. Verfahren zum Zerstäuben und Vernebeln von flüssigen Stoffen, insbesondere zu Inhalationszwecken, gekennzeichnet durch

a) Komprimieren des zu zerstäubenden flüssigen Stoffs mit einem Druck von 300 bis 800 x $10^5$ Pascal, so daß sein Volumen verringert wird,

b) Entlassen des komprimierten flüssigen Stoffs in die normale Atmosphäre mit einem Druck von 1 x $10^5$ Pascal, wodurch der flüssige Stoff infolge seines inneren hohen Drucks explosionsartig in kleinste Teilchen zerrissen wird und

c) Wiederholen der Verfahrensschritte a) und b) vorzugsweise mehrere Male.

2. Verfahren nach Anspruch 1, dadurch gekennzeichnet, daß der flüssige Stoff Vitamine, insbesondere Vitamin A, B, C und/oder E und/oder Lecithin enthält.

3. Verfahren nach Anspruch 2, dadurch gekennzeichnet, daß 0,5 bis 5 Gew.-% Vitamin A und/oder 0,5 bis 5 Gew.-% Vitamin E enthalten sind.

4. Verfahren nach einem der Ansprüche 1 bis 3, dadurch gekennzeichnet, daß der flüssige Stoff eine viskose Flüssigkeit, vorzugsweise Öl ist.

5. Verfahren nach einem der Ansprüche 1 bis 4, dadurch gekennzeichnet, daß der flüssige Stoff ein pflanzliches Öl, vorzugsweise Erdnußöl ist.

6. Verfahren nach Anspruch 1, dadurch gekennzeichnet, daß der flüssige Stoff eine Mischung aus Öl, vorzugsweise Erdnußöl mit 2,5 Gew.-% Vitamin-A-Acetat und 4 Gew.-% Vitamin E-Tocoferol ist.

7. Verfahren nach Anspruch 1, dadurch gekennzeichnet, daß der flüssige Stoff eine Mischung aus Öl, vorzugsweise Erdnußöl mit 1,25 % Vitamin-A-Acetat und 0,5 % Vitamin-E-Tocoferol ist.

8. Verfahren nach einem der Ansprüche 1 bis 3, dadurch gekennzeichnet, daß der flüssige Stoff eine salzige Lösung ist.

9. Verfahren nach einem der Ansprüche 4 bis 7, dadurch gekennzeichnet, daß die Kompression mit einem Druck von 550 x $10^5$ Pascal erfolgt.

10. Verfahren nach Anspruch 8, dadurch gekennzeichnet, daß die Kompression mit einem Druck von 430 x $10^5$ Pascal erfolgt.

11. Verfahren nach einem der Ansprüche 1 bis 10, dadurch gekennzeichnet, daß die erzeugten Teilchen eine Größe von etwa 0,5 bis 10 $\mu m^3$, vorzugsweise etwa 1 $\mu m^3$ aufweisen.

12. Verfahren nach einem der Ansprüche 1 bis 11, dadurch gekennzeichnet, daß die erzeugten Teilchen alle in gleicher Weise elektrisch geladen sind.

13. Verfahren nach einem der Ansprüche 1 bis 12 dadurch gekennzeichnet, daß bei den Verfahrensschritten a) und b) jeweils etwa 0,25 ml des flüssigen Stoffes zerstäubt werden.

14 Vorrichtung zum Zerstäuben und Vernebeln von flüssigen Stoffen, insbesondere zu Inhalationszwecken, gekennzeichnet durch

a) einen Kompressionsraum (10), in dem der zu zerstäubende flüssige Stoff mit einem Druck von 300 bis 800 x $10^5$ Pascal komprimiert wird, so daß sein Volumen verringert wird, und

b) mindestens eine gesteuerte Düse (20) , über die der komprimierte flüssige Stoff aus dem Kompressionsraum (10) explosionsartig in die normale Atmosphäre mit einem Druck von 1 x $10^5$ Pascal entlassen wird, wobei der flüssige Stoff infolge seines inneren hohen Drucks in kleinste Teilchen zerreißt.

15. Vorrichtung nach Anspruch 14, gekennzeichnet durch eine Hochdruckpumpe (30), die über eine Leitung (32) mit dem Kompressionsraum (10) verbunden ist.

16. Vorrichtung nach Anspruch 14 oder 15, dadurch gekennzeichnet, daß die gesteuerte Düse (20) mindestens eine kleine Öffnung (22) und einen Schließer (24) aufweist, der abhängig von einem einstellbaren Druck die Öffnung freigibt.

17. Vorrichtung nach einem der Ansprüche 14 bis 16, dadurch gekennzeichnet, daß die Düse (20) bei einem einstellbaren oberen Druckwert zwischen 300 und 800 x $10^5$ Pascal selbsttätig öffnet und bei einem einstellbaren unteren Druckwert von 200 bis 600 x $10^5$ Pascal selbsttätig schließt.

18. Vorrichtung nach einem der Ansprüche 14 bis 17, dadurch gekennzeichnet, daß die Größe des Kompressionsraumes (10) einstellbar ist.

19. Vorrichtung nach einem der Ansprüche 14 bis 18, dadurch gekennzeichnet, daß die Düse (20) so an einem Nebeltopf (40) angeordnet ist, daß die zerstäubten Teilchen in den Nebeltopf entlassen werden.

20. Vorrichtung nach Anspruch 19, dadurch gekennzeichnet, daß der Nebeltopf (40) eine Zuluftöffnung (42) und eine Auslaßöffnung (48) aufweist.

21. Vorrichtung nach Anspruch 20, dadurch gekennzeichnet, daß die Zuluftöffnung (42) mit einem ersten Schalldämpfer (44) und die Auslaßöffnung (48) mit einem zweiten Schalldämpfer (50) versehen ist.

22. Vorrichtung nach Anspruch 20 oder 21, dadurch gekennzeichnet, daß an der Auslaßöffnung (48) ein Mundstück (60) angeordnet ist.

23. Vorrichtung nach Anspruch 22, dadurch gekennzeichnet, daß das Mundstück (60) ein Einweg-Ventil (62) aufweist, das öffnet, wenn Luft aus dem Nebeltopf (40) angesaugt wird.

FIG. 1

EP 0 377 174 A2